(19) Europäisches Patentamt
European Patent Office
Office européen des brevets

(11) Numéro de publication: **0 208 581**
**B1**

# (12) FASCICULE DE BREVET EUROPEEN

(45) Date de publication du fascicule du brevet: **14.03.90**

(21) Numéro de dépôt: **86401273.7**

(22) Date de dépôt: **11.06.86**

(51) Int. Cl.[5]: **C 07 C 53/10**, C 07 C 53/122, C 01 F 17/00

## (54) Nouveau composé de cérium IV et son procédé de préparation.

(30) Priorité: **20.06.85 FR 8509375**

(43) Date de publication de la demande:
**14.01.87 Bulletin 87/03**

(45) Mention de la délivrance du brevet:
**14.03.90 Bulletin 90/11**

(84) Etats contractants désignés:
**AT BE CH DE FR GB IT LI LU NL SE**

(56) Documents cités:
**FR-A-1 232 937**
**FR-A-2 416 867**
**GB-A-2 102 780**

(73) Titulaire: **RHONE-POULENC CHIMIE**
**25, quai Paul Doumer**
**F-92408 Courbevoie Cédex (FR)**

(72) Inventeur: **Chane-Ching, Jean-Yves**
**19, Raritam Avenue**
**Highland Park NJ 08904 (US)**

(74) Mandataire: **Dutruc-Rosset, Marie-Claude et al**
**RHONE-POULENC INTERSERVICES Service Brevets Chimie 25, Quai Paul Doumer**
**F-92408 Courbevoie Cédex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 208 581 B1

Courier Press, Leamington Spa, England.

## Description

La présente invention concerne un nouveau composé de cérium IV et son procédé d'obtention.

Plus précisément, l'invention a pour but de fournir un composé de cérium IV susceptible d'être aisément dispersable dans l'eau.

Il est connu selon l'Encyclopédie KIRK-OTHMER—Encyclopedia of Chemical Technology (deuxième édition) volume 4, p. 850 que l'on peut préparer un dioxyde cérique hydraté répondant à la formule $CeO_2$, x $H_2O$ dans laquelle x est un nombre compris entre 0,5 et 2 et se présentant sous la forme d'un précipité gélatineux par addition d'hydroxyde de sodium ou d'ammonium aux solutions de sels cériques.

On a proposé selon le brevet français 2 482 075 de préparer un composé de cérium IV dispersable dans l'eau selon un procédé qui consiste à disperser en milieu aqueux, un hydrate d'oxyde de cérium IV sensiblement sec ayant subi un traitement thermique entre 200°C et 450°C en présence d'un agent de désagrégation, notamment l'acide nitrique. Ainsi le chauffage en présence d'un agent de désagrégation réalise la désintégration des cristallites agrégés dans l'hydrate d'oxyde de cérium IV et produit ainsi un composé de cérium dispersable.

Il est mentionné dans ce brevet que la préparation de l'hydrate d'oxyde de cérium (IV) peut se réaliser par précipitation à partir d'un sel de cérium: ainsi par exemple, on peut dissoudre du carbonate céreux de grande pureté dans une solution d'acide nitrique ou chlorhydrique pour obtenir une solution neutre de nitrate ou chlorure céreux que l'on oxyde avec $NH_4OH$/$H_2O_2$ pour obtenir l'hydrate d'oxyde de cérium IV.

Il est également connu, selon GB—A—2 102 780, de faire appel à titre d'agent de désagrégation, à un sel métallique ou un sel d'ammonium.

Il est décrit dans FR—A—2 416 867, un procédé de préparation d'une dispersion aqueuse d'oxyde de cérium qui consiste à former une suspension d'hydroxyde de cérium IV avec de l'eau et un acide mineral ou organique comme l'acide trichloroacétique en une quantité telle que le pH de la suspension soit inférieur à 5,4, à chauffer la suspension pendant une période et à une température telles que le pH soit stable, à ajouter de l'eau pour produire une dispersion aqueuse d'oxyde de cérium.

La demanderesse a trouvé un composé du cérium IV aisément dispersable et qui est obtenu directement à partir de la solution aqueuse d'un sel de cérium IV sans passage par l'étape de précipitation et de séparation de l'hydroxyde cérique.

Le composé de cérium IV selon l'invention répond à la formule générale (I):

$$Ce(M)_t(CH_3—[CH_2]_n—COO^-)_x(OH^-)_y(NO_3^-)_z (I)$$

dans laquelle:

—M représente un métal alcalin ou un radical ammonium quaternaire

—t est compris entre 0,1 et 0,3

—n est égal à 0 ou 1

—x est compris entre 0,1 et 0,7

—y est tel que: $y=4+t-x-z$

—z est compris entre 0,3 et 0,6.

L'analyse par diffraction X montre que le produit de l'invention est un produit mal cristallisé de structure type $CeO_2$.

La présente invention fournit également un procédé de préparation dudit composé caractérisé par le fait qu'il consiste:

—à mélanger une solution aqueuse d'un sel de cérium IV avec de l'acide acétique ou de l'acide propionique, dans un rapport molaire $COO^-$/Ce IV compris entre 0,1 et 15,

—à faire réagir le mélange obtenu avec une base de façon à obtenir un rapport molaire $OH^-$/Ce IV inférior ou égal à 4, et supérieur ou égal à 1, la base étant un hydroxyde alcalin ou l'ammoniac

—à soumettre le mélange réactionnel à un traitement thermique à une température comprise entre 50°C et 100°C

—à séparer le précipité obtenu

—et à le sécher.

Dans la première étape du procédé, on met en oeuvre une solution aqueuse d'un sel de cérium IV et de l'acide acétique ou de l'acide propionique qui peut être en solution aqueuse.

La solution du sel de cérium mise en oeuvre selon la procédé de l'invention est une solution aqueuse de nitrate cérique ou une solution aqueuse de nitrate céri-ammoniacal. Ladite solution peut contenir sans inconvénient du cérium à l'état céreux mais il est souhaitable qu'elle contienne au moins 85% de cérium IV.

La solution de sel de cérium est choisie de telle sorte qu'elle ne contienne pas d'impuretés qui puissent se retrouver dans le produit final. Notamment, il est préférable qu'elle soit exempte d'anions covalents à caractère coagulant tels que sulfates, etc. Cependant, on peut en tolérer de faibles quantités. Par exemple, lesdits anions peuvent représenter jusqu'à 5% en poids du sel de cérium exprimé en $CeO_2$.

La concentration de la solution du sel de cérium n'est pas un facteur critique, selon l'invention. Lorsqu'elle est exprimée en cérium IV, elle peut varier entre 0,1 et 2 moles par litre. Il peut être intéressant pour des questions de productivité d'appareillage, de faire appel à une solution concentrée de sel de cérium IV: une concentration comprise entre 1 et 2 moles per litre est préférée.

La solution aqueuse du sel de cérium IV présente généralement une certaine acidité initiale et peut avoir une normalité variant entre 0,1 N et 4 N. La concentration en ions $H^+$ n'est pas critique. Il est souhaitable qu'elle se situe entre 0,1 N et 1 N.

La solution de nitrate cérique obtenue selon le procédé d'oxydation électrolytique d'une solution de nitrate céreux et qui est décrite dans la demande de brevet française FR—A—2 570 087 (No. 84 13641) constitue une matière première de choix.

Pour ce qui est de l'acide acétique ou de l'acide propionique, on les choisit exempts d'impuretés. Ils peuvent être utilisés indifféremment dilués, par exemple 1 N, ou concentrés. D'une manière préférentielle, on fait appel à l'acide acétique ou l'acide propionique concentré du commerce.

On met en oeuvre de préférence, l'acide acétique à l'acide propionique.

La proportion de l'acide acétique ou l'acide propionique à utiliser par rapport au sel de cérium IV n'est pas critique. Le rapport molaire entre l'acide mis en jeu et le sel de cérium IV exprimé en $CeO_2$ peut varier entre 0,1 et 15 et est choisi de préférence entre 0,5 et 2.

Selon le procédé de l'invention, on effectue le mélange sous agitation de la solution aqueuse du sel de cérium IV et de l'acide acétique ou de l'acide propionique, à une température allant de la température ambiante jusqu'à 80°C. Il est déconseillé de dépasser 80°C afin d'éviter la dégradation de l'acide utilisé.

Dans la deuxième étape du procédé, on additionne une base.

La solution basique mise en oeuvre selon le procédé de l'invention peut être notamment une solution aqueuse d'ammoniaque, de soude ou de potasse. On peut également faire appel à l'ammoniac gazeux. Selon l'invention, on met en oeuvre de préférence une solution d'ammoniaque.

La normalité de la solution basique mise en oeuvre n'est pas un facteur critique selon l'invention, elle peut varier dans de larges limites, par exemple, entre 0,1 et 11 N mais il est préférable, économiquement, de faire appel à des solutions dont la concentration varie entre 5 et 11 N.

La proportion entre la solution basique et la solution de sel de cérium IV doit être telle que le rapport molaire $OH^-$/Ce IV est supérieur ou égal à 1 et inférieur ou égal à 4: $OH^-$ représentant le nombre de moles $OH^-$ apportées par l'addition de la base et CeIV représente le nombre de moles de Ce IV présents dans le milieu réactionnel.

On choisit de préférence un rapport molaire $OH^-$/CeIV supérieur ou égal à 1,5 et inférieur ou égal à 3,5.

La réaction entre le mélange obtenu dans la première étape et la base mise en oeuvre dans les quantités définies précédemment est effectuée à une température qui peut se situer entre 0°C et 60°C mais de préférence à la température ambiante (le plus souvent 15 à 25°C).

La durée de la réaction n'est pas critique et dépend des capacités de l'appareillage. Elle peut varier entre 1 seconde et 20 heures.

On peut réaliser le mélange des réactifs précités selon plusieurs variantes. Par exemple, en peut faire le mélange simultané, sous agitation, de la solution aqueuse du sel de cérium IV contenant l'acide acétique ou l'acide propionique et de la solution basique ou bien additionner, en continu ou en une seule fois, la base dans la solution aqueuse du sel de cérium IV contenant l'acide acétique ou l'acide propionique.

Dans une troisième étape, le mélange réactionnel est traité thermiquement à une température comprise entre 50° et 100°C et de préférence entre 70°C et 90°C. On peut soumettre le mélange réactionnel immédiatement à la température choisie ou à une montée progressive en température.

Les conditions du traitement thermique ne sont pas critiques: il peut être effectué à l'air ou sous atmosphère de gaz inerte. Il n'est pas nécessaire d'agiter au cours de ce traitement.

La durée de ce traitement peut varier dans de larges limites entre 2 et 24 heures, de préférence entre 4 heures et 24 heures.

En fin d'opération, on récupère un précipité solide qui est séparé par les techniques classiques de séparation liquide-solide:filtration, décantation, centrifugation.

Ou soumet le produit obtenu à un séchage qui peut varier entre la température ambiante et 120°C et de préférence de 60°C à 100°C. Cette opération peut être faite à l'air ou sous pression réduite par exemple entre 1 mm (133,32 Pa) et 100 mm de mercure (13 332,2 Pa). La durée de séchage n'est pas critique.

On obtient selon l'invention, le composé de cérium IV avec un rendement pondéral, exprimé en $CeO_2$ par rapport à la quantité de $CeO_2$ apportée par la solution du sel de cérium IV, pouvant varier entre 75 et 98%.

Le composé de l'invention obtenu selon le procédé décrit ci-dessus présente la propriété de former directement une dispersion aqueuse de composé de cérium IV que l'on dénommera par la suite sous le nom de "sol".

Un autre objet de la présente invention réside dans les sols aqueux obtenus à partir du composé de cérium IV répondant à la formule (I).

La présente invention propose également un procédé de préparation d'un sol aqueux d'un composé de cérium IV, procédé selon lequel on met en suspension dans l'eau, le composé de cérium IV répondant à la formule (I).

Pour ce qui est de l'eau, sa nature n'est pas critique et sa température est généralement la température ambiante.

D'une manière préférentielle, on effectue la préparation dudit sol, sous agitation.

Conformément à la présente invention, le composé de cérium IV se trouve sous la forme d'une dispersion colloïdale dans l'eau ce qui signifie que ledit composé a des particules de dimensions colloïdales mais ceci n'exclut pas la présence de Ce IV sous forme ionique.

On peut préparer selon l'invention un sol aqueux d'un composé de cérium IV dont la concentration exprimée en $CeO_2$ peut atteindre jusqu'à 1,5 mole/litre et varie, de préférence, entre 0,2 et 1,5 mole/litre.

On peut obtenir un sol dont la taille des colloïdes peut varier dans une gamme assez large.

La taille des colloïdes est définie par la mesure du diamètre hydrodynamique des colloïdes déterminé par diffusion quasi-élastique de la lumière selon la méthode décrite par Michael L. McConnel dans Analytical Chemistry Vol. 53 No. 8, 1007A (1981): ledit diamètre peut varier entre 10 nm (100 Å) et 100 nm (1000 Å).

Les sols obtenus selon l'invention présentent des propriétés de stabilité au cours du stockage: il n'y a pas de décantation après plusieurs mois de stockage.

On donne ci-après des exemples qui illustrent l'invention sans toutefois la limiter.

Dans les exemples, les pourcentages donnés sont exprimés en poids.

Exemple 1

a) Préparation d'un composé de cérium IV répondant à la formule (I)

Dans un ballon tricol de 2 litres, muni d'un thermomètre, d'un dispositif d'agitation, d'un système d'introduction de réactifs (pompe doseuse), on introduit 100 $cm^3$ d'acide acétique commercial 17,5 N que l'on mélange à température ambiante avec 400 $cm^3$ d'une solution de nitrate cérique contenant 1,23 mole/litre de cérium IV, 0,05 mole/litre de cérium III et ayant une acidité libre de 0,66 N obtenue par électrolyse conformément à FR—A—2 570 087.

Dans ladite solution maintenue sous agitation, on additionne, à température ambiante pendant 5 h 34 mn, une solution d'ammoniaque 3,6 N à raison de 100 $cm^3$ par heure.

En fin d'addition, le mélange réactionnel contient du cérium IV à une concentration exprimée en $CeO_2$ égale à 80 g/l, un rapport molaire $OH^-$/Ce IV voisin de 3,5 est un rapport molaire acide acétique/Ce IV voisin de 3,5.

Dans une deuxième étape, on soumet le mélange réactionnel à un traitement thermique. A cet effet, on met à l'étuve à 80°C le mélange réactionnel précédemment obtenu.

Au bout de 24 heures, on recueille un précipité jaune-blanc par filtration sur verre fritté (porosité No. 3).

On soumet ensuite le produit obtenu à un séchage à l'air réalisé dans une étuve à 80°C pendant environ 24 heures.

On recueille 109,2 g d'un précipité présentant une perte au feu d'environ 25%.

On détermine une rendement de précipitation en cérium égal à 97%.

L'analyse chimique du produit obtenu a conduit aux résultats suivants (les rapports donnés sont des rapports molaires):

—Ce III/Ce IV=0,02
—$NO_3^-$/Ce IV=0,44
—$NH_4^+$/Ce IV=0,17
—acétate/Ce IV=0,29

L'analyse par diffraction X montre que le produit de l'invention est un produit mal cristallisé qui présente une phase cristalline $CeO_2$ de type fluorine.

b)—Préparation du sol aqueux du composé de cérium IV

On additionne 45,86 g du composé préparé selon a) dans de l'eau distillée mise en oeuvre en quantité suffisante pour obtenir un volume de 200 $cm^3$.

On obtient un sol présentant un aspect limpide à l'oeil ayant une concentration en cérium IV exprimée en $CeO_2$ de 172 g/l (1 mole/litre).

L'examen par diffusion quasi-élastique de la lumière montre la présence de colloïdes de diamètre hydrodynamique de l'ordre de 30 nm (300 Å).

On remarque que le sol obtenu présente une bonne stabilité au stockage et ne présente pas de décantation pour une durée d'au moins 1 an.

Exemple 2

a) On reproduit l'exemple 1 à la différence près que l'on met en oeuvre:

—400 $cm^3$ d'une solution de nitrate cérique contenant 1,23 mole/litre de cérium IV, 0,05 mole/litre de cérium III et ayant une acidité libre de 0,66 N

—400 $cm^3$ d'acide acétique concentré 17,5 N

—138 $cm^3$ d'une solution d'ammoniaque 11 N additionnés de 118 $cm^3$ d'eau.

En fin de réaction, le mélange réactionnel contient du cérium IV à une concentration exprimée en $CeO_2$ égale à 80 g/l, un rapport molaire OH/Ce IV voisin de 2,5 et un rapport molaire acide acétique/Ce IV voisin de 14.

Dans une deuxième étape, on soumet le mélange réactionnel à un traitement thermique dans une étuve à 100°C pendant 24 heures.

Après séchage à l'air, on recueille 68,30 g de produit dont l'analyse chimique est la suivante:

—$CeO_2$=70%
—rapport molaire acétate/Ce IV=0,67

b) On prépare le sol comme dans l'exemple 1 par addition de 49,14 g du produit précédemment obtenu dans de l'eau distillée en quantité suffisante pour obtenir un volume de 200 $cm^3$.

On obtient un sol ayant une concentration en cérium IV exprimée en $CeO_2$ de 172 g/l.

L'examen par diffusion quasi-élastique à la lumière effectuée sur une partie aliquote diluée à 0,35 mole/litre de cérium IV met en évidence la présence de colloïdes de diamètre hydrodynamique d'environ 15 nm (150 Å).

Exemple 3

a) On reproduit l'exemple 1 à la différence près que l'on utilisé:

—450 $cm^3$ d'une solution de nitrate cérique contenant 1,23 mole/litre de cérium IV, 0,05 mole/litre de cérium III et ayant une acidité libre de 0,66 N

—50 $cm^3$ acide acétique concentré du commerce à 17,5 N environ

—693 $cm^3$ d'une solution d'ammoniaque 3,14 N.

En fin de réaction, le mélange réactionnel

contient du cérium IV à une concentration exprimée en $CeO_2$ égale à 80 g/l, un rapport molaire $OH^-$/Ce IV voisin de 3,5 et un rapport molaire acide acétique/Ce IV voisin de 1,55.

Dans une deuxième étape ou soumet le mélange réactionnel à un traitement thermique dans une étuve à 100°C pendant 24 heures.

Après séchage à l'air, on recueille 122 g d'un produit contenant 70,5% de $CeO_2$.

b) On prépare le sol comme dans l'exemple 1 par addition de 48,79 g du produit précédemment obtenu dans de l'eau distillée en quantité suffisante pour obtenir un volume de 200 $cm^3$.

On obtient un sol ayant une concentration en cérium IV exprimée en $CeO_2$ de 172 g/l.

L'examen par diffusion quasi-élastique de la lumière effectuée sur une partie aliquote met en évidence la présence de colloïdes ayant un diamètre hydrodynamique d'environ 20 nm (200 Å).

Exemple 4

a) On reproduit l'exemple 1 à la différence près que l'on met en oeuvre:

—500 $cm^3$ d'une solution de nitrate cérique contenant 1,54 mole/litre de cérium IV, 0,08 mole/litre de cérium III et ayant une acidité libre de 0,415 N

—44 $cm^3$ d'acide acétique concentré 17,5 N

—1111,5 $cm^3$ d'une solution d'ammoniaque 2,61 N.

Le mélange réactionnel contient du cérium IV à une concentration exprimée en $CeO_2$ égale à 80 g/l, un rapport molaire $OH^-$/Ce IV voisin de 3,5 et un rapport molaire acide acétique/Ce IV égal à 1.

Dans une deuxième étape, ou soumet le mélange réactionnel à une traitement thermique. A cet effet, on met à l'étuve à 100°C le mélange réactionnel précédemment obtenu.

Au bout de 12 heures, on recueille un précipité par filtration sur verre fritté (porosité No. 4).

On soumet ensuite le produit obtenu à un séchage à l'air réalisé dans une étuve à 80°C pendant environ 24 heures.

On recueille 161,3 g de produit dont l'analyse chimique est la suivante:

—$CeO_2$=75%
—rapport molaire $NO_3^-$/Ce IV=0,53
—rapport molaire $NH_4^+$/Ce IV=0,26
—rapport molaire acétate/Ce IV=0,2

On détermine un rendement de précipitation en cérium égal à 95%.

L'analyse par diffraction X montre que le produit obtenu est mal cristallisé et présente une phase cristalline $CeO_2$: le taux de cristallisation déterminé par rapport à un échantillon témoin est d'environ 35% et la taille des cristallites élémentaires est inférieure à 3 nm (30 Å).

b) On additionne 45,86 g du composé préparé selon a) dans de l'eau distillée mise en oeuvre en quantité suffisante pour obtenir un volume de 200 $cm^3$.

On obtient un sol présentant un aspect limpide à l'oeil ayant une concentration en cérium IV exprimée en $CeO_2$ de 172 g/l (1 mole/litre).

L'examen par diffusion quasi-élastique de la lumière montre la présence de colloïdes de diamètre hydrodynamique de l'ordre de 14 nm (140 Å).

On remarque que le sol obtenu présente une bonne stabilité au stockage et ne présente pas de décantation au cours du temps.

Exemple 5

a) On reproduit l'exemple 1 à la différence près que l'on utilise:

—500 $cm^3$ d'une solution de nitrate cérique contenant 1,54 mole/litre de cérium IV, 0,08 mole/litre de cérium III et ayant une acidité libre de 0,415 N

—57 $cm^3$ d'acide propionique concentré du commerce à 99% (d=0,99 à 1,00)

—1098 $cm^3$ d'une solution d'ammoniaque 1,59 N.

En fin de réaction le mélange réactionnel contient du cérium IV a une concentration exprimée en $CeO_2$ égale à 80 g/l, un rapport molaire $OH^-$/Ce IV voisin de 2 et un rapport molaire acide propionique/Ce IV égal à 1.

Dans une deuxième étape ou soumet le mélange réactionnel à un traitement thermique dans une étuve à 80°C pendant 24 heures.

Après séchage à l'air, on recueille 84,1 g d'un produit contenant 75% de $CeO_2$.

On détermine un rendement de précipitation en cérium égal à 47,6%.

b) On prépare le sol comme dans l'exemple 1 par addition de 45,86 g du produit précédemment obtenu dans de l'eau distillée en quantité suffisante pour obtenir un volume de 200 $cm^3$.

On obtient un sol ayant une concentration en cérium IV exprimée en $CeO_2$ de 172 g/l (1 mole/litre).

L'examen par diffusion quasi-élastique de la lumière, effectuée sur une partie aliquote met en évidence la présence de colloïdes ayant un diamètre hydrodynamique d'environ 34,5 nm (345 Å).

On remarque que le sol obtenu présente une bonne stabilité au stockage et ne présente pas de décantation au cours du temps.

**Revendications**

1. Composé de cérium IV répondant à la formule générale (I):

$$Ce(M)_t(CH_3{-}\!\!\!-[CH_2]_n{-}\!\!\!-COO^-)_x(OH^-)_y(NO_3^-)_z \quad (I)$$

dans laquelle:

—M représente un métal alcalin ou un radical ammonium quaternaire
—t est compris entre 0,1 et 0,3
—n est égal à 0 ou 1
—x est compris entre 0,1 et 0,7
—y est tel que: y=4+t−x−z
—z est compris entre 0,3 et 0,6.

2. Procédé de préparation du composé décrit dans la revendication 1 caractérisé par le fait qu'il consiste:

—à mélanger une solution aqueuse de nitrate cérique ou de nitrate céri-ammoniacal avec de l'acide acétique ou de l'acide propionique dans un rapport molaire $COO^-/CeIV$ compris entre 0,1 et 15

—à faire réagir le mélange obtenu avec une base de façon à obtenir un rapport molaire $OH^-/Ce\ IV$ inférieur ou égal à 4, et supérieur ou égal à 1 la base étant un hydroxyde alcalin ou l'ammoniac

—à soumettre le mélange réactionnel à un traitement thermique à une température comprise entre 50°C et 100°C

—à séparer le précipité obtenu

—et à le sécher.

3. Procédé selon la revendication 2 caractérisé par le fait que la solution aqueuse de nitrate cérique est obtenue par oxydation électrochimique d'une solution aqueuse de nitrate céreux.

4. Procédé selon l'une des revendications 2 et 3 caractérisé par le fait que la concentration en sel de cérium IV exprimée en cérium IV varie entre 0, 1 et 2 moles/litre.

5. Procédé selon la revendication 4 caractérisé par le fait que la concentration en sel de cérium IV exprimée en cérium IV est comprise entre 1 et 2 moles/litre.

6. Procédé selon l'une des revendications 2 à 5 caractérisé par le fait que le rapport molaire entre l'acide acétique ou l'acide propionique et le sel de cérium IV exprimé en $CeO_2$ est compris entre 0,5 et 2.

7. Procédé selon l'une des revendications 2 à 6 caractérisé par le fait que l'on effectue le mélange de la solution aqueuse du sel de cérium IV et de l'acide acétique ou propionique à une température allant de 15°C jusqu'à 80°C.

8. Procédé selon l'une des revendications 2 à 7 caractérisé par le fait que la solution basique est une solution aqueuse d'ammoniaque, de soude, de potasse ou de l'ammoniac gazeux.

9. Procédé selon l'une des revendications 2 à 8 caractérisé par le fait que la normalité de la solution basique est comprise entre 0,1 et 11 N.

10. Procédé selon la revendication 9 caractérisé par le fait que la normalité de la solution basique est comprise entre 5 et 11 N.

11. Procédé selon l'une des revendications 2 à 10 caractérisé par le fait que le rapport molaire $OH^-/Ce\ IV$ est supérieur ou égal à 1 et inférieur ou égal à 4.

12. Procédé selon la revendication 11 caractérisé par le fait que le rapport molaire $OH^-/Ce\ IV$ est supérieur ou égal à 1,5 et inférieur ou égal à 3,5.

13. Procédé selon l'une des revendications 2 à 12 caractérisé par le fait que l'on effectue la réaction entre le mélange obtenu dans la première étape et la base à une température qui se situe entre 0°C et 60°C.

14. Procédé selon la revendication 13 caractérisé par le fait que la température de réaction est choisie entre 15° et 25°C.

15. Procédé selon l'une des revendications 2 à 14 caractérisé par le fait que l'on mélange simultanément, sous agitation, la solution aqueuse du sel de cérium IV contenant l'acide acétique ou l'acide propionique et la solution basique ou bien que l'on additionne, en continu ou en une seule fois, la base dans la solution aqueuse du sel de cérium IV contenant l'acide acétique ou l'acide propionique.

16. Procédé selon l'une des revendications 2 à 15 caractérisé par le fait que la température du traitement thermique est comprise entre 70°C et 90°C.

17. Procédé selon l'une des revendications 2 et 16 caractérisé par le fait que la durée du traitement thermique varie entre 2 et 24 heures.

18. Procédé selon la revendication 17 caractérisé par le fait que la durée dudit traitement thermique est comprise entre 4 et 24 heures.

19. Procédé selon l'une des revendications 2 à 18 caractérisé par le fait que l'on récupère un précipité solide séparé par les techniques classiques de séparation puis soumis à une opération de séchage.

20. Procédé selon la revendication 19 caractérisé par le fait que la température de séchage est comprise entre 15°C et 120°C.

21. Sol aqueux obtenu par mise en dispersion dans l'eau d'un composé de cérium IV selon la revendication 1.

22. Sol aqueux selon la revendication 21 caractérisé par le fait que la concentration du composé de cérium IV exprimée en $CeO_2$ peut varier entre 0,2 et 1,5 mole/litre.

23. Sol aqueux selon l'une des revendications 21 ou 22 caractérisé par le fait que le diamètre hydrodynamique des colloïdes varie entre 10 nm (100 Å) et 100 nm (1000 Å).

**Patentansprüche**

1. Cer-IV-Verbindung entsprechend der allgemeinen Formel (I)

$$Ce(M)_t(CH_3-[CH_2-]_n-COO^-)_x(OH^-)_y(NO_3^-)_z \quad (I)$$

in der

—M ein Alkalimetall oder einen quaternären Ammoniumrest bedeutet,

—t 0,1 bis 0,3 ist,

—n 0 oder 1 ist,

—x 0,1 bis 0,7 ist,

—y so gewählt wird, daß $y=4+t-x-z$ ist, und

—z zwischen 0,3 und 0,6 ist.

2. Verfahren zur Herstellung von Verbindungen nach Anspruch 1, dadurch gekennzeichnet, daß man

—eine wäßrige Lösung von Cer-IV-nitrat oder Cer-IV-ammoniumnitrat mit Essigsäure oder Propionsäure in einem Molverhältnis $COO^-/CeIV$ zwischen 0,1 und 15 vermischt,

—das erhaltene Gemisch mit einer Base derart umsetzt, daß man ein Molverhältnis $OH^-/CeIV$ unter oder gleich 4 und über oder gleich 1 erhält, wobei die Base ein Alkalihydroxid oder Ammoniak ist,

—des Reaktionsgemisch einer thermischen Behandlung bei einer Temperatur zwischen 50 und 100°C unterwirft,

—den erhaltenen Niederschlag abtrennt

—und ihn trocknet.

3. Verfahren nach Anspruch 2, dadurch gekennzeichnet, daß die wäßrige Lösung von Cer-IV-nitrat erhalten worden ist durch elektrochemische Oxidation einer wäßrigen Lösung von Cer-IIInitrat.

4. Verfahren nach einem der Ansprüche 2 oder 3, dadurch gekennzeichnet, daß die Konzentration an Cer-IV-Salz, ausgedrückt als Cer-IV, zwischen 0,1 und 2 mol/l liegt.

5. Verfahren nach Anspruch 4, dadurch gekennzeichnet, daß die Konzentration an Cer-IV-Salz, ausgedrückt als Cer-IV, zwischen 1 und 2 mol/l liegt.

6. Verfahren nach einem der Ansprüche 2 bis 5, dadurch gekennzeichnet, daß das Molverhältnis Essigsäure oder Propionsäure zu Cer-IV-Salz, ausgedrückt als $CeO_2$ zwischen 0,5 und 2 liegt.

7. Verfahren nach einem der Ansprüche 2 bis 6, dadurch gekennzeichnet, daß man das Mischen der wäßrigen Lösung des Cer-IV-Salzes mit der Essigsäure oder Propionsäure bei einer Temperatur durchführt, die von 15 bis auf 80°C geht.

8. Verfahren nach einem der Ansprüche 2 bis 7, dadurch gekennzeichnet, daß die basische Lösung eine wäßrige Ammoniak-, Natriumhydroxid-, Kaliumhydroxidlösung oder gasförmiger Ammoniak ist.

9. Verfahren nach einem der Ansprüche 2 bis 8, dadurch gekennzeichnet, daß die Normalität der basischen Lösung zwischen 0,1 und 11 N liegt.

10. Verfahren nach Anspruch 9, dadurch gekennzeichnet, daß die Normalität der basischen Lösung zwischen 5 und 11 N liegt.

11. Verfahren nach einem der Ansprüche 2 bis 10, dadurch gekennzeichnet, daß das Molverhältnis $OH^-$/CeIV über oder gleich 1 und unter oder gleich 4 ist.

12. Verfahren nach Anspruch 11, dadurch gekennzeichnet, daß das Molverhältnis $OH^-$/CeIV über oder gleich 1,5 und unter oder gleich 3,5 ist.

13. Verfahren nach einem der Ansprüche 2 bis 12, dadurch gekennzeichnet, daß man die Reaktion zwischen dem in der ersten Stufe erhaltenen Gemisch und der Base bei einer Temperatur durchführt, die zwischen 0 und 60°C liegt.

14. Verfahren nach Anspruch 13, dadurch gekennzeichnet, daß die Reaktionstemperatur zwischen 15 und 25°C gewählt wird.

15. Verfahren nach einem der Ansprüche 2 bis 14, dadurch gekennzeichnet, daß man gleichzeitig unter Rühren die wäßrige Lösung des Cer-IV-Salzes, enthaltend die Essigsäure oder die Propionsäure, und die basische Lösung vermischt, oder daß man kontinuierlich oder auf einmal die Base zu der wäßrigen Lösung des Cer-IV-Salzes, enthaltend die Essigsäure oder die Propionsäure, zugibt.

16. Verfahren nach einem der Ansprüche 2 bis 15, dadurch gekennzeichnet, daß die Temperatur der thermischen Behandlung zwischen 70 und 90°C liegt.

17. Verfahren nach einem der Ansprüche 2 bis 16, dadurch gekennzeichnet, daß die Dauer der thermischen Behandlung zwischen 2 und 24 h variiert.

18. Verfahren nach Anspruch 17, dadurch gekennzeichnet, daß die Dauer der thermischen Behandlung zwischen 4 und 24 h liegt.

19. Verfahren nach einem der Ansprüche 2 bis 18, dadurch gekennzeichnet, daß man einen festen abgetrennten Niederschlag nach bekannten Abtrennverfahren gewinnt und anschließend einer Trocknung unterwirft.

20. Verfahren nach Anspruch 19, dadurch gekennzeichnet, daß die Temperatur der Trocknung zwischen 15 und 120°C liegt.

21. Wäßriges Sol, erhalten durch Dispergieren einer Verbindung von Cer-IV, nach Anspruch 1 in Wasser.

22. Wäßriges Sol nach Anspruch 21, dadurch gekennzeichnet, daß die Konzentration an Cer-IV-Verbindung, ausgedrückt als $CeO_2$, zwischen 0,2 und 1,5 mol/l variieren kann.

23. Wäßriges Sol nach einem der Ansprüche 21 oder 22, dadurch gekennzeichnet, daß der hydrodynamische Durchmesser der Kolloide zwischen 10 nm (100 Å) und 100 nm (1000 Å) variiert.

## Claims

1. A compound of cerium IV corresponding to the general formula (I):

$$Ce(M)_t(CH_3-[CH_2]_n-COO^-)_x(OH^-)_y(NO_3^-)_z \quad (I)$$

wherein:

—M represents an alkali metal or a quaternary ammonium radical

—t is between 0.1 and 0.3

—n is equal to 0 or 1

—x is between 0.1 and 0.7

—y is such that: y=4+t−x−z and

—z is between 0.3 and 0.6.

2. A process for preparation of the compound set forth in claim 1 characterised in that it comprises:

—mixing an aqueous solution of ceric nitrate or ceri-ammoniacal nitrate with acetic acid or propionic acid in a $COO^-$/CeIV molar ratio of between 0.1 and 15

—reacting the resulting mixture with a base so as to produce an $OH^-$/CeIV molar ratio which is lower than or equal to 4 and higher than or equal to 1, the base being an alkaline hydroxide or ammonia

—subjecting the reaction mixture to a heat treatment at a temperature of between 50°C and 100°C

—separating the resulting precipitate

—and drying it.

3. A process according to claim 2 characterised in that the aqueous solution of ceric nitrate is obtained by electrochemical oxidation of an aqueous solution of cerous nitrate.

4. A process according to one of claims 2 and 3 characterised in that the level of concentration in respect of cerium IV salt expressed in terms of cerium IV varies between 0.1 and 2 moles per litre.

5. A process according to claim 4 characterised in that the level of concentration in respect of cerium IV salt expressed in terms of cerium IV is between 1 and 2 moles per litre.

6. A process according to one of claims 2 to 5 characterised in that the molar ratio between acetic acid or proprionic acid and the cerium IV salt expressed in terms of $CeO_2$ is between 0.5 and 2.

7. A process according to one of claims 2 to 6 characterised by effecting mixing of the aqueous solution of the cerium IV salt and the acetic or propionic acid at a temperature ranging from 15°C to 80°C.

8. A process according to one of claims 2 to 7 characterised in that the basic solution is an aqueous solution of ammonia, sodium hydroxide, potassium hydroxide or gaseous ammonia.

9. A process according to one of claims 2 to 8 characterised in that the normality of the basic solution is between 0.1 and 11 N.

10. A process according to claim 9 characterised in that the normality of the basic solution is between 5 and 11 N.

11. A process according to one of claims 2 to 10 characterised in that the $OH^-$/CeIV molar ratio is higher than or equal to 1 and lower than or equal to 4.

12. A process according to claim 11 characterised in that the $OH^-$/CeIV molar ratio is higher than or equal to 1.5 and lower than or equal to 3.5.

13. A process according to one of claims 2 to 12 characterised by effecting the reaction between the mixture obtained in the first step and the base at a temperature which is between 0°C and 60°C.

14. A process according to claim 13 characterised in that the reaction temperature is between 15°C and 25°C.

15. A process according to one of claims 2 to 14 characterised by simultaneously mixing with agitation the aqueous solution of the cerium IV salt containing acetic acid or propionic acid and the basic solution or adding the base continuously or in a single step to the aqueous solution of the cerium IV salt containing the acetic or propionic acid.

16. A process according to one of claims 2 to 15 characterised in that the temperature of the heat treatment is between 70°C and 90°C.

17. A process according to one of claims 2 and 16 characterised in that the duration of the heat treatment varies between 2 and 24 hours.

18. A process according to claim 17 characterised in that the duration of said heat treatment is between 4 and 24 hours.

19. A process according to one of claims 2 to 18 characterised by recovering a solid precipitate which is separated by conventional separation procedures and then subjected to a drying operation.

20. A process according to claim 19 characterised in that the drying temperature is between 15°C and 120°C.

21. An aqueous sol obtained by dispersion in water of a compound of cerium IV according to claim 1.

22. An aqueous sol according to claim 21 characterised in that the concentration of the cerium IV compound expressed in terms of $CeO_2$ may vary between 0.2 and 1.5 mole per litre.

23. An aqueous sol according to one of claims 21 and 22 characterised in that the hydrodynamic diameter of the colloids varies between 10 nm (100 Å) and 100 nm (1000 Å).